# EUROPEAN PATENT APPLICATION

(11) **EP 2 143 457 A1**
(43) Date of publication of application: **13.01.2010**
(21) Application number: 09164778.4
(22) Date of filing: 07.07.2009
(51) Int. Cl.: A61M 15/00

(54) **Inhaler**

(30) Priority: 08.07.2008 JP 2008177548
(71) Applicant: Canon Kabushiki Kaisha, Tokyo 146-8501 (JP)
(72) Inventor: Tsuchiya, Satoshi, Tokyo 146-8501 (JP); Nobutani, Toshiyuki, Tokyo 146-8501 (JP); Wada, Hiroyuki, Tokyo 146-8501 (JP); Sugita, Masaru, Tokyo 146-8501 (JP); Kobayashi, Masaya, Tokyo 146-8501 (JP); Sugiyama, Takahiro, Tokyo 146-8501 (JP)
(74) Representative: TBK-Patent

(57) **Abstract**

An inhaler includes an inhaler body 2 having an ejection head 1 and an airflow duct forming member 4 for forming an airflow duct on the inhaler body 2. In an inhalation standby status, the airflow duct forming member 4 is driven to slide so as to be placed at rest on the inhaler body 2 to partly cover the top part of the latter by means of a slide mechanism 8 and make the airflow duct disappear. At this time, the top wall of the airflow duct forming member 4 covers the ejection head 1 to shield it from external air. In an inhalation status, the airflow duct forming member 4 is driven to slide up and produce an airflow duct on the inhaler body 2. Thus, a compact inhaler that can be switched from an inhalation status to an inhalation standby status and vice versa with ease is realized.

## Description

### BACKGROUND OF THE INVENTION

### Field of the Invention

The present invention relates to an inhaler for ejecting liquid as droplets and having a user inhale it.

### Description of the Related Art

Inhalers for ejecting a medicine as micro-droplets into an airflow duct where air flows so as to be inhaled by way of a mouthpiece, utilizing the ejection principle of ink-jet system, and having a user inhale it have been developed (refer to, e.g., International Publication No. WO95/01137). Such inhalers provide an advantage that a predetermined dose of medicine can be accurately administered and sprayed as uniformized droplets.

To date, inhalers having two different caps including a cap for protecting the medicine ejecting section of the inhaler in an inhalation standby status and a mouthpiece cap for protecting the airflow duct in an inhalation standby status have been proposed in order to realize inhalers showing a high operation reliability (see U.S. Patent Application Publication No. 2004/0150690 and Japanese Patent Application Laid-Open No. 2004-283244).

However, the above-described conventional art requires that two caps are handled or a mouthpiece is handled when switching from an inhalation status to an inhalation standby status (where the inhaler is not used) of operation and vice versa to make the overall operation a complex one. Therefore, if the user needs to manually operate for the switching, the operation reliability of ejection of a liquid medicine can be damaged by the risk of operation error that is not negligible. When a mechanism for automatically switching from an inhalation status to an inhalation standby status is installed in the inhaler, the inhaler becomes structurally complex to by turn give rise to a problem of missing the easy portability.

### SUMMARY OF THE INVENTION

In view of the above-identified problems, the object of the present invention is to provide an in haler where an inhalation status and an inhalation standby status can be selected with an easy switching operation and that shows a high operation reliability and an easy portability.

According to the present invention, the above object is achieved by providing an inhaler for ejecting a medicine and having a user inhale it through an inhalation port, including: an inhaler body having a medicine ejecting section for ejecting a medicine; an airflow duct forming member for forming an airflow duct on or in the inhaler body to lead the medicine ejected from the medicine ejecting section to the inhalation port; and a slide mechanism and/or a rotation mechanism for placing the airflow duct forming member at rest on or in the inhaler body in an not-in-use status, so that the space of the airflow duct disappears, wherein the airflow duct forming member is adapted to protect the medicine ejecting section as a result of the operation of being placed at rest.

Thus, according to the present invention, the airflow duct forming member is driven to slide and/or rotate so as to switch from an inhalation status to an inhalation standby status (not-in-use status) and vice versa, which is a simple operation so that the inhaler can be made to show a simple structure. Additionally, the medicine ejecting section is protected in an interlocked manner to allow the user to operate the inhaler highly conveniently.

Furthermore, the size of the inhaler can be reduced in an inhalation standby status than in an inhalation status by the space required to produce the airflow duct.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

FIGS. 1A and 1B are schematic perspective views of the inhaler of Example 1. FIG. 1A is a view of the inhaler in an inhalation status and FIG. 1B is a view of the inhaler in an inhalation standby status.

FIGS. 2A, 2B and 2C are schematic perspective views of the inhaler of Example 1. FIG. 2A is a view of the inhaler from which the airflow duct forming member is taken out, FIG. 2B is a view of the inhaler from which the ejection head and the medicine tank are taken away, and FIG. 2C is a schematic view of the inhaler of which the cover is opened.

FIGS. 3A and 3B are schematic cross sectional views of the inhaler of Example 1 taken along the slide mechanism thereof. FIG. 3A is a view in an inhalation status, and FIG. 3B is a view in an inhalation standby status.

FIGS. 4A and 4B are schematic cross sectional views of the slide mechanism of the inhaler of Example 1. FIG. 4A is a view in an inhalation status, and FIG. 4B is a view in an inhalation standby status.

FIGS. 5A and 5B are schematic views of the inhaler of Example 2. FIG. 5A is a schematic perspective view of the inhaler from which the airflow duct forming member is taken out, and FIG. 5B is a schematic cross sectional view of the inhaler in which the airflow duct forming member is placed at rest.

FIGS. 6A and 6B are schematic views of the inhaler of Example 3. FIG. 6A is a schematic perspective view of the inhaler in an inhalation status, and FIG. 6B is a partially exploded schematic perspective view of the inhaler to illustrate the internal configuration thereof.

FIGS. 7A, 7B and 7C are schematic perspective views of the inhaler of Example 3, illustrating the operation steps taken for shifting from an inhalation status to an inhalation standby status.

FIGS. 8A and 8B are schematic views of the inhaler of Example 3. FIG. 8A is a plan view thereof, and FIG. 8B is a schematic cross sectional view taken along the airflow duct forming member thereof.

FIGS. 9A and 9B are schematic views of the inhaler of Example 3 in an inhalation standby status. FIG. 9A is a plan view thereof, and FIG. 9B is a schematic cross sectional view taken along the airflow duct forming member thereof.

FIGS. 10A and 10B are schematic views of a variation of the inhaler of Example 3. FIG. 10A is a schematic perspective view of the inhaler in an inhalation status, and FIG. 10B is a schematic perspective view of the inhaler in an inhalation standby status.

### DESCRIPTION OF THE EMBODIMENTS

Preferred embodiments of the present invention will now be described in detail in accordance with the accompanying drawings.

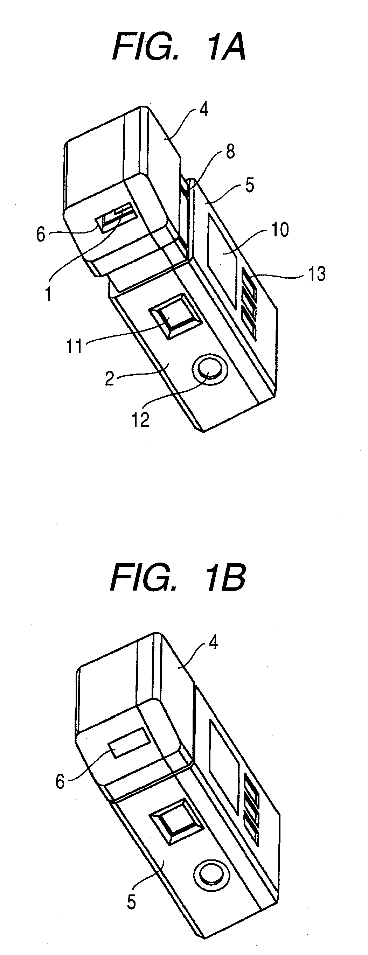

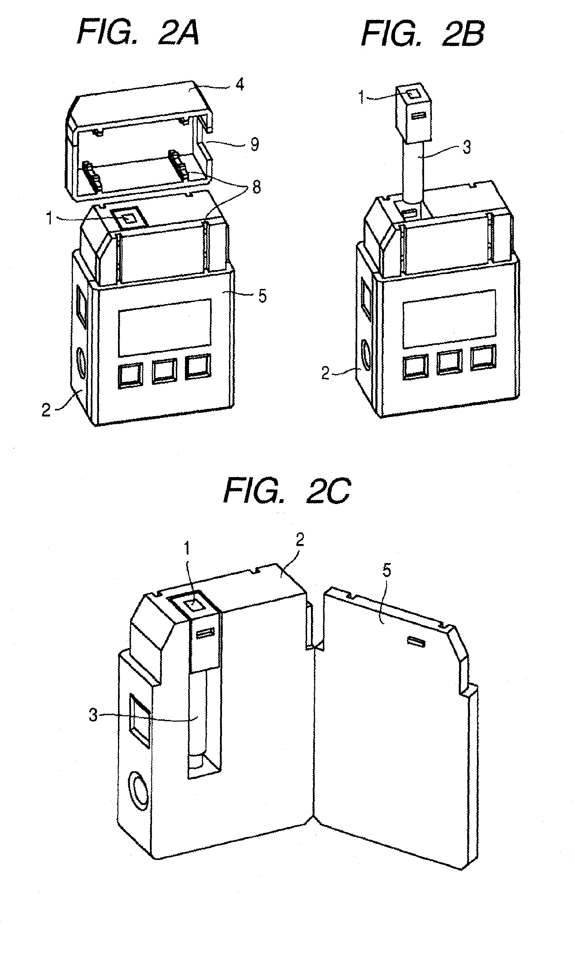

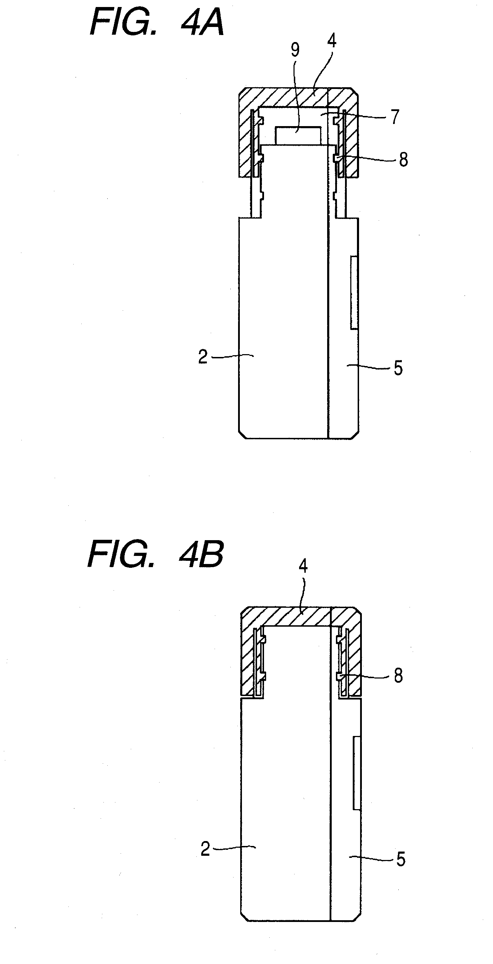

FIGS. 1A and 1B through FIGS. 4A and 4B illustrate an embodiment of the present invention. As illustrated in FIGS. 1A and 1B, this embodiment is so designed that the user thereof carries with him or her in order to inhale a medicine from it. It has an inhaler body 2 including an ejection head 1 that is an ink-jet type medicine ejecting section adapted to eject a medicine as micro-droplets of highly uniform particle size by a predetermined quantity. As illustrated in FIGS. 2A through 2C, a medicine tank 3 that contains a medicine and the ejection head 1 having a plurality of ejection ports for ejecting the medicine supplied from the medicine tank 3 as micro-droplets constitute a cartridge that can be removably fitted to the inhaler body 2.

The ejection head 1 and the medicine tank 3 are so designed that they can be used for a plurality of inhalations. The cartridge of the ejection head 1 and the medicine tank 3 is replaced by a new one when the quantity of medicine in the medicine tank falls short of the dose for a single inhalation. For example, the inhaler body may be provided with a feature of counting the ejections and hence recording the quantity of medicine that has been ejected and the residual quantity of medicine in the medicine tank 3 can be computed by means of the ejection counting feature. Therefore, it may be so arranged as to notify the user of the time of replacement and prompt the user to replace the cartridge or prohibit any ejection until the cartridge is replaced.

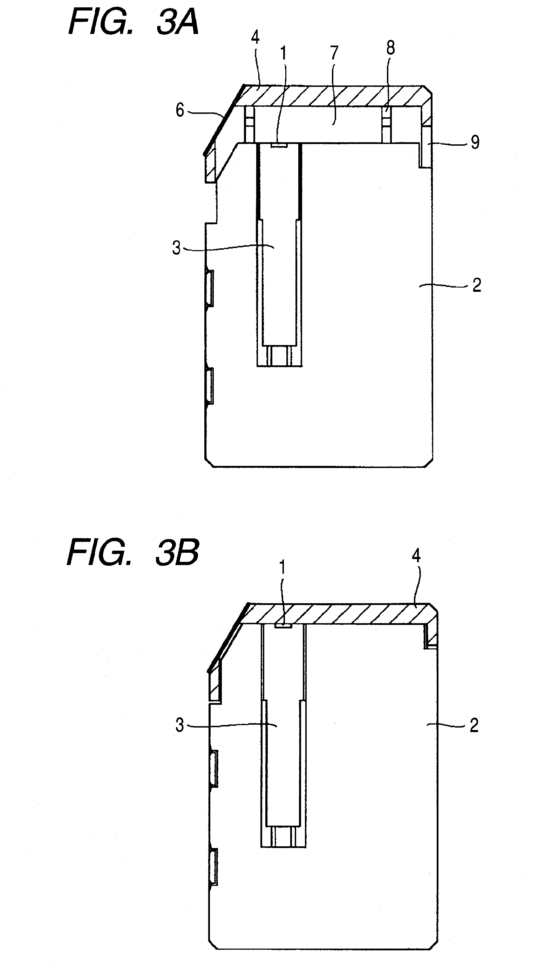

As illustrated in FIGS. 3A and 3B and FIGS. 4A and 4B, the airflow duct forming member 4 produces an airflow duct 7 in the inhaler body. The airflow duct 7 is a space through which the air flow produced by an inhaling action performed by the user in order to lead the medicine ejected from the ejection head 1 to the inhalation port 6. An inhaler according to the present invention has a slide mechanism 8 that operates as guide rails for allowing the airflow duct forming member 4 to be placed on an upper part (airflow duct receiving section) of the inhaler body 2. When the inhaler is not in use, the airflow duct forming member 4 is driven to slide relative to the inhaler body 2 so as to be placed at rest. Then, as a result, the space of the airflow duct 7 disappears and the inner wall of the airflow duct 7 covers and protects the ejection head 1. In other words, the plane of the ejection ports of the ejection head 1 is so arranged as to face the inner wall of the airflow duct forming member 4 and allow the ejection head 1 to eject the medicine into the airflow duct 7. Since the ejection head 1 is so arranged as to face the inner wall surface of the airflow duct forming member 4, the inner wall of the airflow duct 7 can protect the ejection head when the space of the airflow duct 7 is made to disappear by means of the slide mechanism 8. It should be noted, however, that the position of the ejection head 1 is not limited to that of this embodiment. In other words, the ejection head 1 may be arranged at any position where it is protected by the inner wall of the airflow duct when the airflow duct forming member 4 is placed at rest relative to the inhaler body and the space of the airflow duct 7 is made to disappear.

Thus, the user can switch the status of the inhaler from an inhalation status to an inhalation standby status and vice versa simply by driving the airflow duct forming member 4 to slide.

An inhaler according to the present invention may alternatively be so arranged that a part of the airflow duct forming member is driven to slide onto the inhaler body and the remaining part of the airflow duct forming member is driven to rotate so as to come at rest on the medicine ejecting section instead of the arrangement where the entire airflow duct forming member is driven to slide and come into engagement with an upper part of the inhaler body. Still alternatively, it may be so arranged that entire airflow duct forming member is driven to rotate and come at rest on the inhaler body by means of an arched slide mechanism. Still alternatively, it may be so arranged that the airflow duct forming member is simply folded up by means of a rotation mechanism and placed at rest on the inhaler body without providing a slide mechanism as in the case of an example that will be described hereinafter. The airflow duct 7 can be made to disappear with any of these arrangements. Additionally, each of the embodiments that are described herein is **characterized in that** the medicine ejecting section is protected by the inner wall surface of the airflow duct forming member 4 as a result of the operation of placing the airflow duct forming member 4 at rest and making the airflow duct 7 disappear. Thus, a compact inhaler that is held in a satisfactorily sanitary condition when it is not in use can be realized with such a simple arrangement.

The slide mechanism may be so designed that the user directly drive it to slide/rotate or the inhaler may additionally be provided with a drive means for driving it slide/rotate.

Example 1

FIGS. 1A and 1B are schematic perspective views of the inhaler of Example 1. FIG. 1A is a view of the inhaler in an inhalation status (in-use status) and FIG. 1B is a view of the inhaler in an inhalation standby status (not-in-use status). The apparatus has an ink-jet type ejection head 1 that is a medicine ejecting section, an inhaler body 2, an airflow duct forming member 4, a cover 5, an inhalation port 6, a slide mechanism 8, a display section 10, an ejection switch 11, a power switch 12 and selection switches 13. The inhalation port 6 is arranged at an end of the airflow duct forming member 4.

FIG. 2A is a perspective view of the inhaler body 2 and the airflow duct forming member 4 that are separated from each other by means of the slide mechanism 8. The airflow duct forming member 4 includes an air introduction port (air intake port) 9 for introducing external air into the inhaler body 2 when the user inhales the medicine. The ejection head 1 is provided with a plurality of ejection ports. For the purpose of the present invention, ejection energy for ejecting micro-droplets of medicine can be generated by means of a thermal ink-jet system using an electrothermal transducer, a piezoelectric system using an electromechanical transducer or an ejection system using a nebulizer.

FIG. 2B is a perspective view of the inhaler body 2 from which the ejection head 1 is taken out. The medicine tank 3 is integral with the ejection head 1 and held in communication with the ejection head 1 so as to supply a medicine to the ejection head 1. FIG. 2C is a schematic perspective view of the inhaler where the ejection head 1 and the medicine tank 3 are set in position in the inhaler body 2 and the cover 5 of the inhaler body 2 is opened.

FIG. 3A is a cross sectional view of the airflow duct forming member 4 in an inhalation status and FIG. 3B is a cross sectional view of the airflow duct forming member 4 in an inhalation standby status.

Now, the process to be followed by the user to inhale a medicine from the inhaler will be described below. Firstly, the user supplies power to the inhaler by depressing the power switch 12. Then, the user operates the desired one of the selection switches 13 to select a desired medicine ejection mode. Subsequently, the user drives the inhaler to eject the medicine from the ejection head 1 by depressing the ejection switch 11. Then, the user can inhale the medicine from the inhalation port 6. Air flows through the air introduction port 9, the airflow duct 7 and the inhalation port 6 sequentially in the above mentioned order.

As the user ends the inhalation, he or she drives the airflow duct forming member 4 to slide and come into engagement with an upper part of the inhaler body 2 by means of the slide mechanism 8 and makes the airflow duct 7 on the inhaler body disappear. As a result, the inhalation port 6 and the air introduction port 9 are closed by the side of the inhaler body 2.

Then, as a result, both the inhalation port 6 and the air introduction port 9 are closed to prevent dusts and microbes from entering the inside of the inhaler through the airflow duct 7. Additionally, as the upper wall of the airflow duct forming member 4 is brought into tight contact with the ejection head 1, the medicine is prevented from evaporating and escaping through the ejection ports of the ejection head 1.

A recessed section may preferably be formed between the ejection head 1 and the airflow duct forming member 4 in order to prevent the ejection ports from being crushed depending on the relationship between the material of the ejection head 1 that forms the ejection ports and the material of the airflow duct forming member 4.

The user can remove the airflow duct forming member 4 from the inhaler body 2 as illustrated in FIG. 2A to wash the inner wall of the airflow duct 7.

FIG. 4A is a schematic cross sectional view of the slide mechanism 8 of the inhaler of Example 1 in an inhalation status and FIG. 4B is a schematic cross sectional view of the slide mechanism 8 in an inhalation standby status. The slide mechanism 8 has two recesses at the side of the inhaler body 2 and two projections at the side of the airflow duct forming member 4 that can be received respectively by the two recesses for mutual engagement. The airflow duct forming member 4 is placed at the right position both in an inhalation status and in an inhalation standby status by the combinations of the recesses and the projections. The distance between the two recesses and the distance between the two projections are made equal to the height of the airflow duct 7 and about 1 cm to 2 cm.

### Example 2

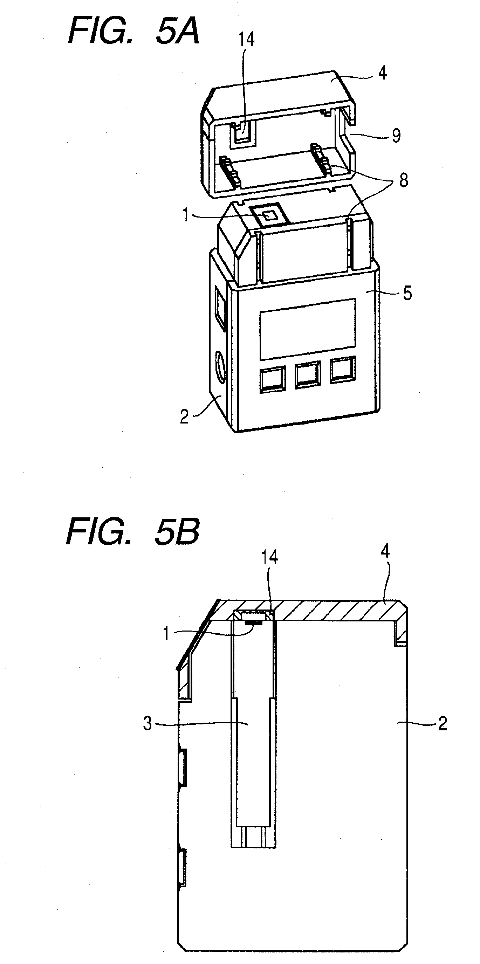

As illustrated in FIGS. 5A and 5B, the inhaler of this example has a cap 14 in addition to the components same as those of the inhaler of Example 1 in order to effectively protect the ejection head 1. The inhaler of this example is particularly effective when the ejection ports of the ejection head 1 are formed by a soft material such as resin.

FIG. 5A is a schematic perspective view of the inhaler body 2 separated from the airflow duct forming member 4. A cap 14 is arranged at the top wall of the airflow duct forming member 4 in order to protect the ejection head 1. The cap 14 is arranged on the inner wall of the airflow duct at a position opposite to the ejection head 1 so that it tightly covers the ejection head 1 as the airflow duct forming member 4 is driven to side by means of the slide mechanism 8. The cap 14 is made of rubber and has a recess at the center thereof. While the cap 14 is arranged on the inner wall of the airflow duct at a position opposite to the ejection head 1 because the airflow duct forming member 4 of the inhaler of this example is driven to slide up and down by means of the slide mechanism, the position of the cap 14 is not limited thereto for the purpose of the present invention. In other words, it is sufficient that the cap 14 that is a head protection member is so arranged as to protect the ejection head when the space of the airflow duct is made to disappear.

With the above-described arrangement, the ejection ports and their vicinities are not brought into direct contact with the rubber of the cap and the cap 14 can tightly close the ejection head 1 as the rubber is deformed when it is brought into contact with the ejection head 1. Thus, the ejection ports are protected against being damaged because they and their vicinities are not brought into direct contact with the cap 14 and additionally, the medicine is prevented from evaporating from the ejection ports. Then, as a result, this example provides advantages including that the ejection characteristics of the inhaler are prevented from being modified due to crushed ejection ports, adhesion of medicine at and near the ejection ports and/or degeneration of the medicine in the ejection ports and that the administration effect is prevented from being degraded.

Example 3

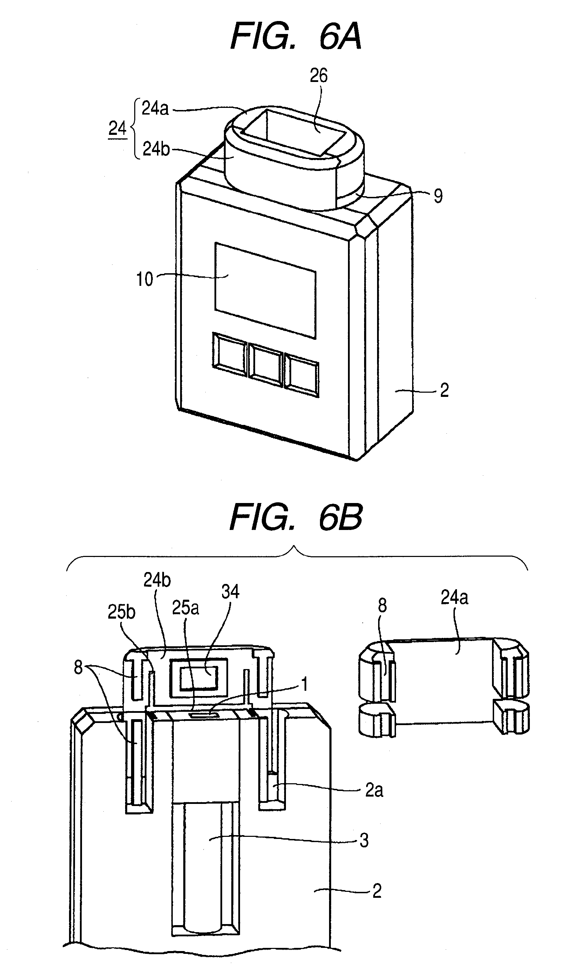

FIGS. 6A and 6B are schematic views of the inhaler of Example 3. As illustrated in FIG. 6A, the inhaler of this example has an airflow duct forming member 24 having an inhalation port 26 with its aperture flush with the top surface of the ejection head 1. As illustrated in FIG. 6B, the airflow duct forming member 24 is divided into a sliding section 24a that is to be contained in the airflow duct containing section 2a in the inhaler body and a rotating section 24b to be driven to rotate onto the ejection head in order to protect the ejection head.

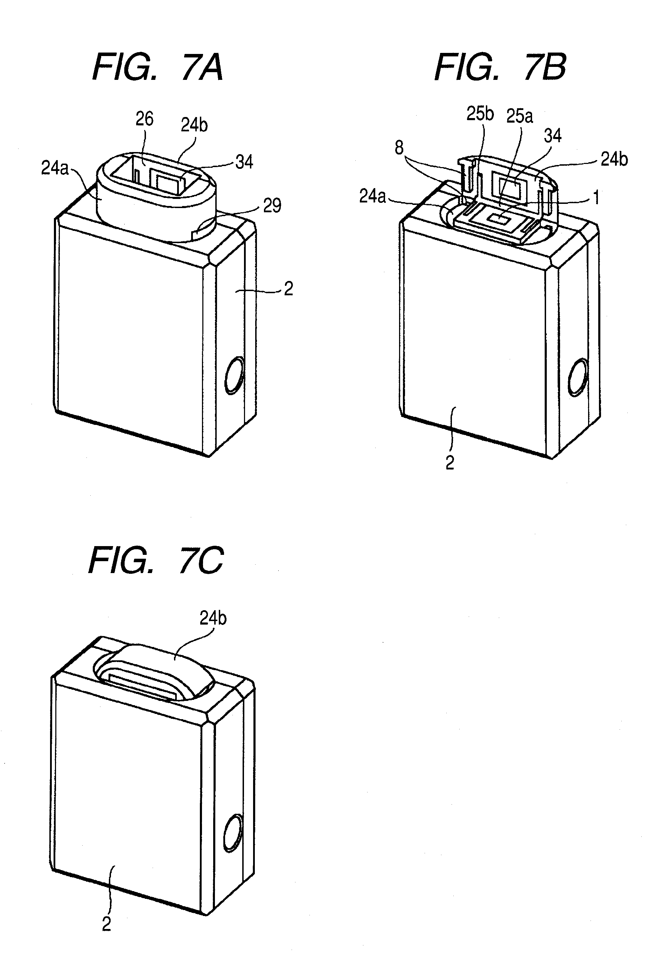

FIGS. 7A through 7C are schematic perspective views of the inhaler of Example 3, illustrating the operation steps taken for shifting the position of the airflow duct forming member 24 from an inhalation status to an inhalation standby status. FIG. 7A is a schematic perspective view of the inhaler of this example in an inhalation status, FIG. 7B is a schematic perspective view of the inhaler being shifted from an inhalation status to an inhalation standby status and FIG. 7C is a schematic perspective view of the inhaler in an inhalation standby status. Since the ejection head 1, the inhaler body 2, the slide mechanism 8 and some other components are the same as those of the inhaler of Example 1, they are denoted respectively by the same reference symbols and will not be described here repeatedly.

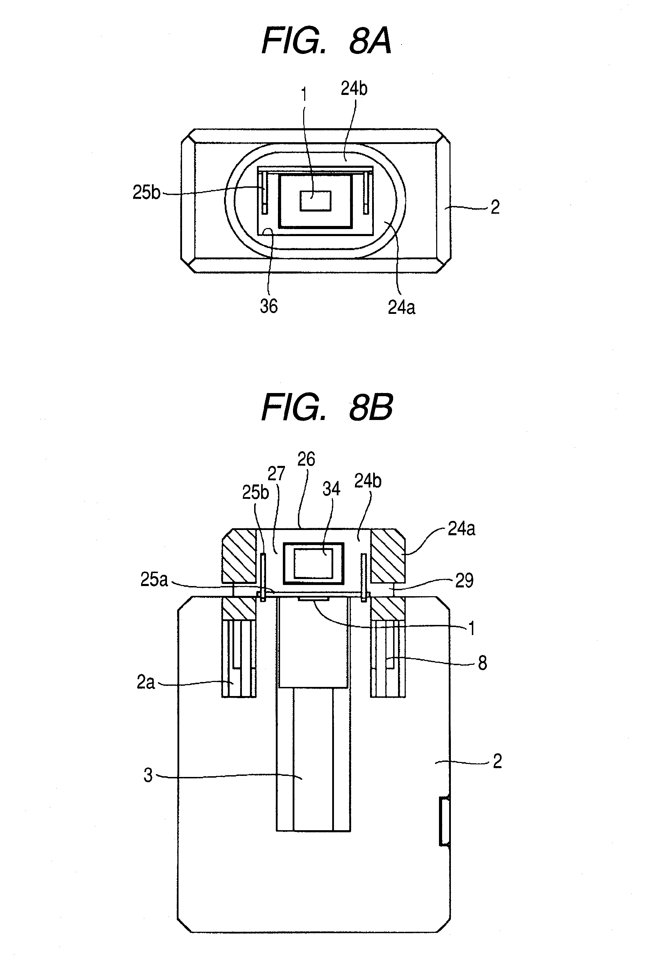

As illustrated in FIG. 7A and FIGS. 8A and 8B, the airflow duct 27 of the inhaler is formed by the sliding section 24a and the rotating section 24b. The user inhales the medicine ejected from the ejection head 1 by way of the inhalation port 26. At this time, air flows through an air introduction port 29, the airflow duct 27 and the inhalation port 26 sequentially in the above mentioned order.

As the user ends the inhalation, he or she contains the airflow duct forming member 24 by shifting its state from the one illustrated in FIG. 7A to the one illustrated in FIG. 7C by way of the one illustrated in FIG. 7B.

Firstly, the user completely contains the sliding section 24a in the airflow duct containing section 2a in the inhaler body by means of the slide mechanism 8 as illustrated in FIG. 7B. The slide mechanism 8 has two projections at the rotating section 24b of the airflow duct forming member 24 and also two projections in the airflow duct containing section 2a of the inhaler body 2, which projections are to be engaged with the corresponding recesses formed on the sliding section 24a. As the projections and the recesses are mutually engaged, the sliding section 24a can be driven to slide up and down. The projections of the rotating section 24b are provided with respective ribs arranged near the suction port 26 in order to prevent the sliding section 24a from coming off. The depth of the airflow duct containing section 2a of the inhaler body 2 is greater than the height of the sliding section 24a so as to completely contain the sliding section 24a in the inhaler body.

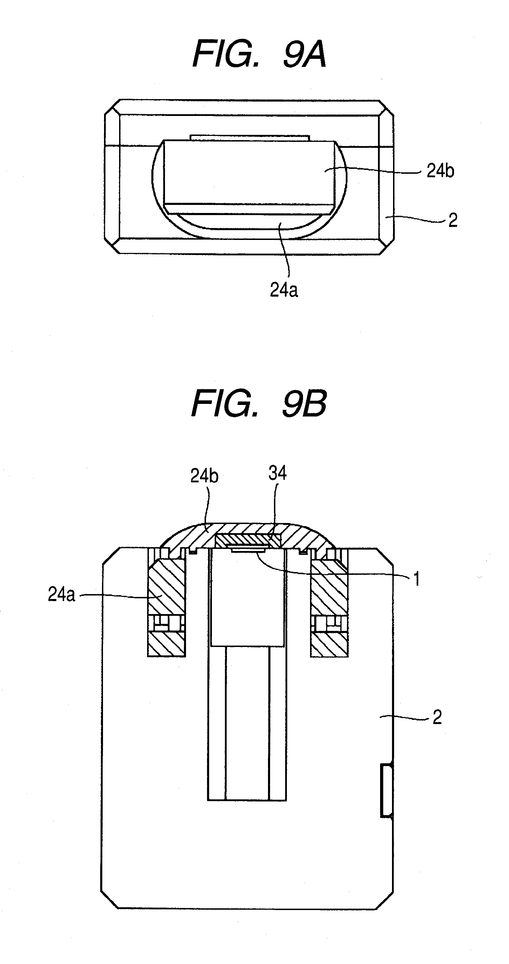

Then, the rotating section 24b is turned toward the inhaler body 2 along a rotation mechanism 25a that operates like the axis of a hinge. A spring 25b is provided between the rotating section 24b and the inhaler body 2 so as to urge the rotating section 24b to get into the state illustrated in FIG. 7C that is more stable than the state illustrated in FIG. 7A and the state illustrated in FIG. 7B. Thus, as a result, the rotating section 24b that is supported by the sliding section 24a in the state illustrated in FIG. 7A and in the state illustrated in FIGS. 8A and 8B automatically turns to get into the stable state illustrated in FIG. 7C and FIGS. 9A and 9B due to the force of the spring 25b simply as the user pushes the sliding section 24a into the airflow duct containing section 2a.

As illustrated in FIGS. 9A and 9B, the cap 34 is arranged at the rotating section 24b so as to cover the ejection head 1. The sliding section 24a is contained in the inhaler body 2 as the rotating section 24b is pushed against the latter by the force of the spring 25b.

In the state illustrated in FIGS. 9A and 9B, the inhalation port 26 and the airflow duct 27 disappear and the air introduction port 29 is closed as it is contained in the inhaler body 2 to prevent dusts and microbes from entering the inside of the apparatus through the airflow duct 27. Additionally, as the ejection head 1 is covered by the cap 34, the medicine is prevented from evaporating and escaping through the ejection ports of the ejection head 1 and the ejection head 1 is prevented from being damaged. Then, as a result, this example provides advantages including that the ejection characteristics of the inhaler are prevented from being modified due to crushed ejection ports, adhesion of medicine at and near the ejection ports and/or degeneration of the medicine in the ejection ports and that the administration effect is prevented from being degraded. While a rotating motion takes place after a sliding motion in the above description, the present invention is by no means limited thereto and, alternatively, a sliding motion may be made to take place after protecting the ejection head 1 by way of a rotating motion so as to make the airflow duct completely disappear.

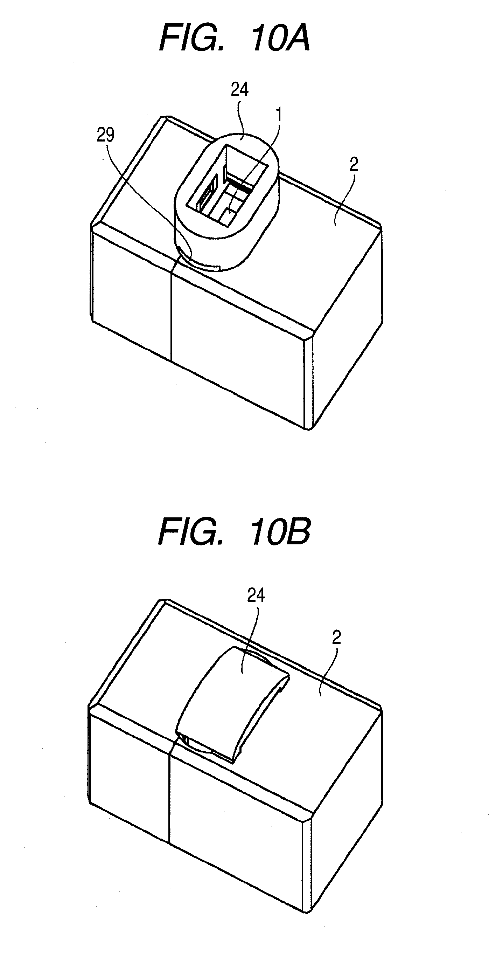

While the airflow duct forming member 24 of this example is divided and a part thereof has a slide mechanism and the remaining part thereof has a rotation mechanism in the above description, the airflow duct forming member 24 may alternatively be realized as a single and inseparable unit as illustrated in FIGS. 10A and 10B. Then, the entire airflow duct forming member 24 is driven to rotate and slide to get into the inhaler body so as to be contained there. With such an arrangement, the slide mechanism and the airflow duct containing section show a circular profile centered at the axis of the rotation mechanism.

Still alternatively, the airflow duct forming member may be divided into a plurality of parts, each of which has a rotation mechanism. With such an arrangement, all the parts of the divided airflow duct forming member are folded up so as to partially cover the top of the inhaler body. Then, no slide mechanism is required and the airflow duct forming member is folded up by the rotation mechanism and put on the inhaler body so as to partially cover the top of the inhaler body. The ejection head is protected by the part of the airflow duct forming member that is folded first. If a cap is provided to protect the ejection head, it is arranged at the part of the divided airflow duct forming member that is to be folded first.

The present invention is not limited to the above embodiments and various changes and modifications can be made within the spirit and scope of the present invention. Therefore, to apprise the public of the scope of the present invention, the following claims are made.

An inhaler includes an inhaler body 2 having an ejection head 1 and an airflow duct forming member 4 for forming an airflow duct on the inhaler body 2. In an inhalation standby status, the airflow duct forming member 4 is driven to slide so as to be placed at rest on the inhaler body 2 to partly cover the top part of the latter by means of a slide mechanism 8 and make the airflow duct disappear. At this time, the top wall of the airflow duct forming member 4 covers the ejection head 1 to shield it from external air. In an inhalation status, the airflow duct forming member 4 is driven to slide up and produce an airflow duct on the inhaler body 2. Thus, a compact inhaler that can be switched from an inhalation status to an inhalation standby status and vice versa with ease is realized.

## Claims

1. An inhaler for ejecting a medicine and having a user inhale it through an inhalation port comprising:
an inhaler body having a medicine ejecting section for ejecting a medicine;
an airflow duct forming member for forming an airflow duct on or in the inhaler body to lead the medicine ejected from the medicine ejecting section to the inhalation port; and
a slide mechanism and/or a rotation mechanism for placing the airflow duct forming member at rest on or in the inhaler body in an not-in-use status, so that the space of the airflow duct disappears, wherein
the airflow duct forming member is adapted to protect the medicine ejecting section as a result of the operation of being placed at rest.

2. The inhaler according to claim 1, wherein
the airflow duct forming member has a cap for protecting the medicine ejecting section.

3. The inhaler according to claims 1 or 2, wherein
the inhalation port arranged at an end of the airflow duct forming member is closed as a result of the operation of placing the airflow duct forming member at rest.

4. The inhaler according to any one of claims 1 to 3, wherein
an air intake port is provided for introducing external air into the airflow duct for a user at the time of inhaling the medicine and designed so as to be closed as a result of the operation of placing the airflow duct forming member at rest.

5. The inhaler according to any one of claims 1 to 4, wherein
the airflow duct forming member has a sliding section to be driven to slide relative to the inhalation body and a rotating section to be driven to rotate relative to the inhaler body.

6. The inhaler according to claim 5, wherein
a cap is arranged at the rotating section to protect the medicine ejecting section.
